(19) Europäisches Patentamt

European Patent Office

Office européen des brevets

(11) Veröffentlichungsnummer: **0 264 924 B1**

(12) **EUROPÄISCHE PATENTSCHRIFT**

(45) Veröffentlichungstag der Patentschrift: **29.01.92**

(21) Anmeldenummer: **87115380.5**

(22) Anmeldetag: **21.10.87**

(51) Int. Cl.⁵: **C07C 49/603**, C07C 45/74, C07C 45/82, C07C 45/85, C07C 45/78

(54) Verfahren zur Erhöhung der Ausbeute an 2,5,6-trimethylcyclohexen-2-on-1.

(30) Priorität: **23.10.86 DE 3636057**

(43) Veröffentlichungstag der Anmeldung:
**27.04.88 Patentblatt 88/17**

(45) Bekanntmachung des Hinweises auf die
Patenterteilung:
**29.01.92 Patentblatt 92/05**

(84) Benannte Vertragsstaaten:
**CH DE FR GB LI**

(56) Entgegenhaltungen:
**DE-C- 1 668 874**
**FR-A- 2 328 685**

(73) Patentinhaber: **BASF Aktiengesellschaft**
**Carl-Bosch-Strasse 38**
**W-6700 Ludwigshafen(DE)**

(72) Erfinder: **Tavs, Peter, Dr.**
**Neuhofener Strasse 45**
**W-6703 Limburgerhof(DE)**
Erfinder: **Laas, Harald, Dr.**
**Scholstrasse 105**
**W-6701 Maxdorf(DE)**
Erfinder: **Schauer, Horst**
**Pfalzring 212**
**W-6704 Mutterstadt(DE)**
Erfinder: **Arnold, Lothar, Dr.**
**Hausackerweg 11**
**W-6900 Heidelberg(DE)**

# EP 0 264 924 B1

**Beschreibung**

Die Erfindung betrifft ein Verfahren zur Erhöhung der Ausbeute an 2,5,6-Trimethylcyclohexen-2-on-1. Die Verbindung kann zum 2,3,6-Trimethylphenol dehydriert werden. Das 2,3,6-Trimethylphenol ist ein wichtiges Vorprodukt für die Synthese von Vitamin E.

In der DE-C-17 93 037 ist ein Verfahren zur Herstellung von 2,5,6-Trimethylcyclohexen-2-on-1 durch Umsetzung von Diethylketon in Gegenwart von Alkali mit Crotonaldehyd oder einer Verbindung, die unter den gewählten Reaktionsbedingungen in Crotonaldehyd umgewandelt wird und destillative Aufarbeitung des Reaktionsgemischs beschrieben. Die Ausbeuten des bekannten Verfahrens lassen jedoch noch zu wünschen übrig, sie liegen ausweislich der Beispiele zwischen 40 und 77 %.

Der Erfindung liegt die Aufgabe zugrunde, ein Verfahren zur Erhöhung der Ausbeute an 2,5,6-Trimethylcyclohexen-2-on-1 mit den Merkmalen des Oberbegriffs des Anspruchs 1 zur Verfügung zu stellen.

Diese Aufgabe wird gelöst mit einem Verfahren der eingangs genannten Art, das dadurch gekennzeichnet ist, daß man den anfallenden Destillationsrückstand entweder

a) unter Zusatz katalytischer Mengen Alkali auf 120 bis 250° C, insbesondere 120 bis 200° C, erhitzt, oder

b) ohne einen solchen Zusatz auf 150 bis 250° C erhitzt

und das aus dem Destillationsrückstand durch die Wärmebehandlung gebildete Gemisch aus 2,5,6-Trimethylcyclohexen-2-on-1 und Diethylketon destillativ abtrennt.

Nach einer bevorzugten Ausführungsform setzt man 0,5 bis 5 Gew.% Kalilauge, bezogen auf den Destillationsrückstand, zu.

Nach einer weiteren bevorzugten Ausführungsform führt man die Wärmebehandlung bei Normaldruck durch und destilliert das gebildete Diethylketon unter Normaldruck und anschließend das 2,5,6-Trimethylcyclohexen-2-on-1 unter vermindertem Druck ab. Wenn man unter vermindertem Druck destilliert, ist ein Bereich von 20 bis 100 mbar besonders zweckmäßig.

Nach einer anderen bevorzugten Ausführungsform führt man die Wärmebehandlung unter vermindertem Druck durch, wobei das gebildete Gemisch aus Diethylketon und 2,5,6-Trimethylcyclohexen-2-on-1 unter vermindertem Druck abdestilliert.

Die Spaltung des Destillationsrückstandes kann sowohl diskontinuierlich wie auch kontinuierlich durchgeführt werden. Bei kontinuierlicher Durchführung ist es bevorzugt, die Wärmebehandlung in einer Reaktionskolonne auszuführen, wobei man den Destillationsrückstand dem unteren Teil der Kolonne oder dem Sumpf zupumpt und das durch Spaltungsreaktion im Sumpf gebildete Gemisch aus 2,5,6-Trimethylcyclohexen-2-on-1 und Diethylketon über den Kolonnenkopf abzieht.

Nach einer weiteren bevorzugten Ausführungsform kann man alternativ die Wärmebehandlung in einem Rührkessel bei einer Verweilzeit von insbesondere 0,5 bis 3 Stunden durchführen, wobei das Diethylketon und ein kleiner Anteil an 2,5,6-Trimethylcyclohexen-2-on-1 abdestillieren und nachfolgend die Hauptmenge des 2,5,6-Trimethylcyclohexen-2-on-1 in einer Destillationskolonne von höhersiedenden Produkten abgetrennt wird.

Wenn man die Spaltung bei Temperaturen von 200 bis 250° C, insbesondere bei Temperaturen oberhalb 220° C durchführt, kann es zweckmäßig sein, zum Destillationsrückstand 5 bis 20 Gew.%, bezogen auf den Destillationsrückstand, einer inerten, hochsiedenden Mineralölfraktion zuzusetzen. Hierdurch wird die Pumpfähigkeit des hochsiedenden Rückstands nach der Spaltungsreaktion aufrecht erhalten. Als Mineralölfraktion kann man beispielsweise Solventöl einsetzen.

An sich wäre zu erwarten gewesen, daß durch die erfindungsgemäße Wärmebehandlung des Destillationsrückstandes dieser verharzt oder unerwünschte Zersetzungsprodukte ergibt. Es wurde jedoch überraschenderweise festgestellt, daß im Destillationsrückstand eine Gruppe von Verbindungen mit einem Molekulargewicht von 224 vorliegt, welche unter den erfindungsgemäßen Bedingungen sich thermisch oder katalytisch in 2,5,6-Trimethylcyclohexen-2-on-1 und Diethylketon rückspalten lassen.

Nach dem erfindungsgemäßen Verfahren kann man aus einer Tonne Destillationsrückstand 120 bis 180 kg 2,5,6-Trimethylcyclohexen-2-on-1 und 85 bis 110 kg Diethylketon gewinnen. Das bei der Zersetzung erhaltene Destillat besteht zu 82 bis 91 % aus den genannten Wertprodukten, die dann durch eine einfache Destillation getrennt werden können.

Beim erfindungsgemäßen Verfahren geht man im allgemeinen von Diethylketon und Crotonaldehyd aus. Man kann aber auch anstelle des Crotonaldehyds Verbindungen einsetzen, die unter geeigneten Reaktionsbedingungen in Crotonaldehyd umgewandelt werden. Beispiele hierfür sind $\beta$-Hydroxybutyraldehyd und $\beta$-Methoxibutyraldehyd. Im allgemeinen setzt man das Diethylketon im Überschuß ein. Bei der destillativen Aufarbeitung erhält man als erste Fraktion den Überschuß des eingesetzten Diethylketons und als zweite

2

Fraktion das Wertprodukt. Es verbleibt der Destillationsrückstand, der nach den Maßnahmen des erfindungsgemäßen Verfahrens aufgearbeitet wird. Alternativ kann man als dritte Fraktion eine höhersiedende Fraktion abziehen, die dann in gleicher Weise wie der Destillationsrückstand erfindungsgemäß weiterverarbeitet wird.

Die Erfindung wird durch die nachfolgenden Beispiele weiter erläutert.

Der in den nachstehenden Beispielen eingesetzte Destillationsrückstand wurde wie in der DE-C-17 93 037 beschrieben erhalten.

Beispiel 1
Spaltung mit Kalilauge

1200 g des Destillationsrückstandes und 15 g 50 %ige Kalilauge werden in eine Destillationsapparatur eingefüllt. Die Apparatur wird evakuiert (ca. 20 Torr) und das Gemisch wird innerhalb 75 Minuten von 70° C auf 175° C erwärmt. Die Destillationsvorlage wird mit Eiswasser gekühlt. Es destilliert 136 g fast farbloses Produkt über. Im Destillat sind (GC-Analyse) 70,5 g 2,5,6-Trimethylcyclohexen-2-on-1 (TMCH) und 42,2 g Diethylketon (DEK) enthalten.

Zersetzung des Destillationsrückstandes von TMCH mit Kalilauge (Versuchsbedingungen wie vorstehend)

| Destillationsrückstand | KOH 50 % | Destillat | |
|---|---|---|---|
| | | TMCH | DEK |
| g | g | g | g |
| 1200 | – | 68,2 | 36,9 |
| 1200 | 15 | 70,3 | 42,1 |
| 1200 | 30 | 126,7 | 66,7 |
| 1200 | 60 | 174,9 | 103,9 |

Beispiel 2
Spaltung mit Kalilauge bei Normaldruck, Destillation im Vakuum

In einem 800 l Rührkessel werden 418 kg Destillationsrückstand und 21 kg 50 %ige KOH eingefüllt. Der Kesselinhalt wird bei Normaldruck auf 170° C erhitzt und 2 Stunden bei dieser Temperatur gehalten. Es destillieren insgesamt 44,9 kg Produkt, welches sich in 2 Schichten trennt. Die untere Phase besteht aus 12 kg Wasser, welches abgetrennt wird. Danach wird evakuiert und zuletzt bei 45 Torr insgesamt 82,1 kg Destillat erhalten. Insgesamt werden 115 kg organisches Produkt erhalten. Darin sind laut GC-Analyse 65,13 kg TMCH und 37,84 kg DEK enthalten.

Beispiel 3
Spaltung ohne Kalilauge

In einer Destillationsapparatur mit aufgesetzter Kolonne (40×2,5 cm) werden 500 g Destillationsrückstand auf 248° C (Ölbad) erhitzt. Es destilliert zunächst DEK und wenig TMCH ab. Nach 70 Minuten wird die Sumpftemperatur auf 160° C abgesenkt und durch allmähliche Evakuierung der Destillationsapparatur das gebildete TMCH übergetrieben.

Insgesamt werden 158,1 g farblose Flüssigkeit erhalten, die laut GC-Analyse 85,7 g TMCH und 50,2 g DEK enthalten. Der Destillationsrückstand ist nach dem Erkalten zähflüssig.

Beispiel 4

Beispiel 3 wird unter Zusatz von 50 g Solventöl wiederholt. Es werden 156,9 g Destillat gesammelt, welches 86,0 g TMCH und 47,4 g DEK enthält. Der Destillationsrückstand dieses Versuches ist nach dem Erkalten dünnflüssig.

**Patentansprüche**

1. Verfahren zur Erhöhung der Ausbeute an 2,5,6-Trimethylcyclohexen-2-on-1 durch Umsetzung von Diethylketon in Gegenwart von Alkali mit Crotonaldehyd oder einer Verbindung, die unter den gewählten Reaktionsbedingungen in Crotonaldehyd umgewandelt wird und destillative Aufarbeitung des Reaktionsgemischs, dadurch gekennzeichnet, daß man den anfallenden Destillationsrückstand entweder
   a) unter Zusatz katalytischer Mengen Alkali auf 120 bis 250 ° C, insbesondere 120 bis 200 ° C, erhitzt, oder
   b) ohne einen solchen Zusatz auf 150 bis 250 ° C erhitzt
   und das aus dem Destillationsrückstand durch die Wärmebehandlung gebildete Gemisch aus 2,5,6-Trimethylcyclohexen-2-on-1 und Diethylketon destillativ abtrennt.

2. Verfahren nach Anspruch 1, dadurch gekennzeichnet, daß man 0,5 bis 5 Gew.%, bezogen auf den Destillationsrückstand, an Kalilauge zusetzt.

3. Verfahren nach einem der Ansprüche 1 oder 2, dadurch gekennzeichnet, daß man die Wärmebehandlung bei Normaldruck durchführt, das gebildete Diethylketon unter Normaldruck und anschließend das 2,5,6-Trimethylcyclohexen-2-on-1 unter vermindertem Druck abdestilliert.

4. Verfahren nach einem der Ansprüche 1 oder 2, dadurch gekennzeichnet, daß man die Wärmebehandlung unter vermindertem Druck durchführt und gleichzeitig das gebildete Gemisch aus Diethylketon und 2,5,6-Trimethylcyclohexen-2-on-1 unter vermindertem Druck abdestilliert.

5. Verfahren nach einem der Ansprüche 1 bis 4, dadurch gekennzeichnet, daß man die Wärmebehandlung kontinuierlich in einer Reaktionskolonne durchführt, wobei man den Destillationsrückstand dem unteren Teil der Kolonne oder dem Sumpf zupumpt und das durch Spaltungsreaktion im Sumpf gebildete Gemisch aus 2,5,6-Trimethylcyclohexen-2-on-1 und Diethylketon über den Kolonnenkopf abzieht.

6. Verfahren nach einem der Ansprüche 1 bis 4, dadurch gekennzeichnet, daß man die Wärmebehandlung in einem Rührkessel bei einer Verweilzeit von 0,5 bis 3 Stunden durchführt und nachfolgend in einer Destillationskolonne das 2,5,6-Trimethylcyclohexen-2-on-1 von höhersiedenden Produkten abtrennt.

7. Verfahren nach einem der Ansprüche 1 bis 6, dadurch gekennzeichnet, daß man die Wärmebehandlung bei Temperaturen von 200 bis 250 ° C durchführt, wobei man dem Destillationsrückstand 5 bis 20 Gew.%, bezogen auf den Destillationsrückstand, einer inerten, hochsiedenden Mineralölfraktion zusetzt.

**Claims**

1. A process for increasing the yield of 2,5,6-trimethylcyclohex-2-en-1-one by reacting diethyl ketone, in the presence of an alkali, with crotonaldehyde or a compound which is converted to crotonaldehyde under the reaction conditions chosen, and working up the reaction mixture by distillation, wherein the distillation residue obtained is either
   a) heated at from 210 to 250 ° C, in particular from 120 to 200 ° C, with the addition of a catalytic amount of an alkali, or
   b) heated at from 150 to 250 ° C without the addition of such a substance,
   and the mixture of 2,5,6-trimethylcyclohex-2-en-1-one and diethyl ketone which is formed from the distillation residue as a result of the heat treatment is separated by distillation.

2. A process as claimed in claim 1, wherein from 0.5 to 5% by weight, based on the distillation residue, of potassium hydroxide solution is added.

3. A process as claimed in one of claims 1 and 2, wherein the heat treatment is carried out under atmospheric pressure, the diethyl ketone formed is distilled off under atmospheric pressure and the 2,5,6-trimethylcyclohex-2-en-1-one is distilled off under reduced pressure.

4. A process as claimed in one of claims 1 and 2, wherein the heat treatment is carried out under reduced pressure and at the same time the resulting mixture of diethyl ketone and 2,5,6-trimethylcyclohex-2-en-1-one is distilled off under reduced pressure.

5. A process as claimed in one of claims 1 to 4, wherein the heat treatment is carried out continuously in a reaction column, the distillation residue being pumped to the lower part of the column or to the bottom, and the mixture of 2,5,6-trimethylcyclohex-2-en-1-one and diethyl ketone which is formed by the cleavage reaction in the bottom being taken off at the top of the column.

6. A process as claimed in one of claims 1 to 4, wherein the heat treatment is carried out in a stirred kettle during a residence time of from 0.5 to 3 hours, and subsequently the 2,5,6-trimethylcyclohex-2-en-1-one is separated from higher boiling products in a distillation column.

7. A process as claimed in one of claims 1 to 6, wherein heat treatment is carried out at from 200 to 250°C, from 5 to 20% by weight, based on the distillation residue, of an inert, high boiling mineral oil fraction being added to the distillation residue.

**Revendications**

1. Procédé pour augmenter le rendement en 2,5,6-triméthylcyclohexène-2-one-1 obtenue par réaction de la diéthylcétone en présence d'alcali avec l'aldéhyde crotonique ou un composé qui est transformé en aldéhyde crotonique dans les conditions choisies pour la réaction et par traitement subséquent par distillation du mélange réactionnel, caractérisé en ce que l'on chauffe le résidu de distillation obtenu
   a) soit avec une addition de quantités catalytiques d'alcali à une température de 120 à 250°C, en particulier 120 à 200°C,
   b) soit sans une telle addition à une température de 150 à 250°C
   et en ce que l'on sépare par distillation le mélange de 2,5,6-triméthylcyclohexène-2-one-1 et de diéthylcétone formé à partir dudit résidu de distillilation par le traitement par la chaleur.

2. Procédé suivant la revendication 1, caractérisé en ce que l'on ajoute 0,5 à 5% en poids de lessive de potasse par rapport au résidu de distillation.

3. Procédé suivant la revendication 1 ou 2, caractérisé en ce que l'on effectue à la pression normale le traitement par la chaleur, et en ce que l'on sépare par distillation la diéthylcétone formée sous la pression normale puis la 2,5,6-triméthylcyclohexène-2-one-1 sous pression réduite.

4. Procédé suivant la revendication 1 ou 2, caractérisé en ce que l'on effectue le traitement par la chaleur sous pression réduite et en ce que l'on sépare par distillation sous pression réduite simultanément le mélange formé de diéthylcétone et de 2,5,6-triméthylcyclohexène-2-one-1.

5. Procédé suivant l'une quelconque des revendications 1 à 4, caractérisé en ce que l'on effectue le traitement par la chaleur en continu dans une colonne de réaction, en amenant par pompage le résidu de distillation dans la partie inférieure ou le fond de la colonne et en retirant en tête de colonne le mélange de 2,5,6-triméthylcyclohexène-2-one-1 et de diéthylcétone formé au fond de la colonne par une réaction de dissociation.

6. Procédé suivant l'une quelconque des revendications 1 à 4, caractérisé en ce que l'on effectue le traitement par la chaleur dans une cuve pourvue d'un agitateur avec un temps de séjour de 0,5 à 3 heures et en ce que l'on sépare ensuite, dans une colonne de distillation, la 2,5,6-triméthylcyclohexène-2-one-1 des produits ayant un point d'ébullition plus élevé.

7. Procédé suivant l'une quelconque des revendications 1 à 6, caractérisé en ce que l'on effectue le traitement par la chaleur à des températures de 200 à 250°C, en ajoutant au résidu de distillation 5 à 20% en poids par rapport au résidu de distillation, d'une fraction d'huile minérale inerte, de haut point d'ébullition.